# EUROPÄISCHE PATENTSCHRIFT

(11) **EP 2 445 545 B1**
(45) Veröffentlichungstag und Bekanntmachung des Hinweises auf die Patenterteilung: **13.08.2014**
(21) Anmeldenummer: 10718535.7
(22) Anmeldetag: 23.04.2010
(51) Int. Cl.: A61M 1/10, A61M 1/36, F04D 15/00

(54) **VORRICHTUNG ZUR FÖRDERUNG VON BLUT IN EINEM EXTRAKORPORALEN KREISLAUF**
DEVICE FOR PUMPING BLOOD IN AN EXTRACORPOREAL CIRCUIT
DISPOSITIF DE CIRCULATION DU SANG DANS UN CIRCUIT EXTRACORPOREL

(30) Priorität: 25.06.2009 DE 102009027195
(43) Veröffentlichungstag der Anmeldung: 02.05.2012
(73) Patentinhaber: Sorin Group Deutschland GmbH, 80939 München (DE)
(72) Erfinder: PENKA, Ottmar, 80997 München (DE); SCHREYER, Johann, 80999 München (DE); KNOTT, Erwin, 85586 Poing (DE)
(74) Vertreter: HOFFMANN EITLE
(86) Internationale Anmeldenummer: PCT/EP2010/055444
(87) Internationale Veröffentlichungsnummer: WO 2010/149408

(56) Entgegenhaltungen:
- DE-A1- 19 840 399
- US-A- 6 048 363
- US-A1- 2003 045 772
- US-B1- 6 564 627

## Beschreibung

Die Erfindung betrifft eine Vorrichtung zur Förderung von Blut in einem extrakorporalen Kreislauf mit einer Zentrifugalpumpe.

Die Förderung von Blut in einem extrakorporalen Blutkreislauf mit einer Zentrifugalpumpe ist bekannt, beispielsweise aus DE 39 35 502 A1 und aus DE 19 840 399. Bei diesen Vorrichtungen führt eine venöse Schlauchleitung das Blut vom Patienten an die Pumpe heran und eine arterielle Schlauchleitung von der Pumpe weg. Da bei diesen Vorrichtungen die Zentrifugalpumpe unmittelbar an die venöse Zuflussleitung angeschlossen ist, kann es bei zu großer Ansaugleistung der Pumpe zu einem übermäßig hohen Unterdruck in der venösen Schlauchleitung kommen, der zu einer Schädigung des Blutes führt. Um dies zu vermeiden, kann auf der Ansaugseite der Pumpe der Unterdruck mit Hilfe eines Sensors erfasst werden, um beim Erreichen eines vorgegebenen Wertes geeignete Gegenmaßnahmen gegen einen übermäßig hohen Unterdruck auszulösen, etwa durch Alarmierung einer Bedienperson oder einen automatischen Eingriff in den Pumpenbetrieb.

Jedoch ist die Erfassung des Unterdrucks auf der venösen Seite der Pumpe problematisch, da es wegen des hier herrschenden Unterdrucks (gegenüber dem Atmosphärendruck) zum Eindringen von Luft in den Blutkreislauf kommen kann, wenn am Ort des Drucksensors Undichtigkeiten auftreten. Die Erkennung von eindringender Luft aufgrund dieser Problematik ist praktisch nicht durchführbar; gleichzeitig stellt Luft, die in den extrakorporalen Blutkreislauf gelangt, eine erhebliche Bedrohung für den Patienten dar.

Das der Erfindung zugrunde liegende Problem besteht vor diesem Hintergrund darin, einen Weg anzugeben, wie ein zuverlässiger Wert, der der Höhe des Unterdrucks auf der Ansaugseite der Pumpe entspricht, erhalten werden kann, ohne dazu einen Drucksensor auf der venösen Seite der Pumpe einzusetzen.

Gelöst wird dieses Problem durch eine Vorrichtung zur Förderung von Blut in einem extrakorporalen Kreislauf mit einer Zentrifugalpumpe, die das zu fördernde Blut über eine an der Ansaugseite der Pumpe angeschlossene venöse Schlauchleitung ansaugt und über eine an der Abgabeseite der Pumpe angeschlossene arterielle Schlauchleitung abgibt, mit einem Durchflusssensor, der zur Erfassung der Abgabemenge auf der Abgabeseite der Pumpe vorgesehen ist und der ein der Abgabemenge entsprechendes Messsignal abgibt, mit einem Drucksensor, der zur Erfassung des Druckes auf der Abgabeseite der Pumpe vorgesehen ist und der ein dem Druck entsprechendes Messsignal abgibt, und mit einer Auswerteeinrichtung, der das Messsignal des Durchflusssensors und das Messsignal des Drucksensors zugeführt werden und die unter Einbeziehung der beiden Messsignale und der Drehzahl der Zentrifugalpumpe einen dem Druck auf der Ansaugseite der Pumpe entsprechenden Wert ermittelt.

In einer vorteilhaften Ausgestaltung nutzt die Auswerteeinrichtung bei der Ermittlung des dem Druck auf der Ansaugseite der Pumpe entsprechenden Werts ein Kennlinienfeld der Zentrifugalpumpe.

In einer weiteren vorteilhaften Ausgestaltung ist in der Auswerteeinrichtung das Kennlinienfeld der Zentrifugalpumpe in einem Speicher abgelegt.

In einer weiteren vorteilhaften Ausgestaltung ist die Auswerteeinrichtung mit einer Anzeigeeinrichtung für die Anzeige zumindest des Unterdrucks auf der Ansaugseite der Pumpe und/oder für die Alarmierung einer Bedienperson verbunden.

In einer weiteren vorteilhaften Ausgestaltung wird der Auswerteeinrichtung ein Messsignal eines Drehzahlsensors zugeführt wird.

In einer weiteren vorteilhaften Ausgestaltung wird der Auswerteeinrichtung ein Ansteuersignal der Pumpe zugeführt, das die Drehzahl der Pumpe bestimmt bzw. repräsentiert.

In einer weiteren vorteilhaften Ausgestaltung nimmt die Auswerteeinrichtung die Funktion einer Steuereinrichtung für die Zentrifugalpumpe wahr oder ist in eine Steuereinrichtung für die Zentrifugalpumpe integriert, so dass ein die Drehzahl der Zentrifugalpumpe bestimmendes Ansteuersignal der Zentrifugalpumpe zuführbar ist.

In einer weiteren vorteilhaften Ausgestaltung reduziert die Auswerteeinrichtung die Drehzahl der Pumpe beim Auftreten eines vorgegebenen Wertes für den Druck auf der Ansaugseite der Pumpe.

Im Folgenden wird ein Ausführungsbeispiel der Erfindung unter Bezugnahme auf die Zeichnung genauer beschrieben.

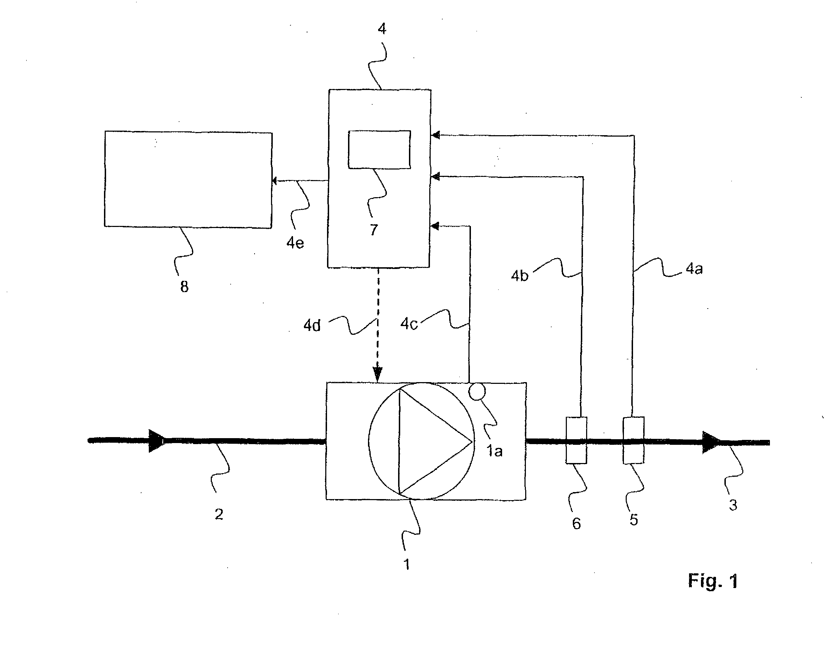

- Figur 1: zeigt den Aufbau eines Ausführungsbeispiels einer erfindungsgemäßen Vorrichtung.

Das in Figur 1 gezeigte Ausführungsbeispiel einer erfindungsgemäßen Vorrichtung zur Förderung von Blut in einem extrakorporalen Kreislauf umfasst eine Zentrifugalpumpe 1, die das zu fördernde Blut über eine an der Ansaugseite der Pumpe angeschlossene venöse Schlauchleitung 2 ansaugt. Die venöse Schlauchleitung 2 kann unmittelbar mit einem Patienten verbunden sein, dessen Blut in dem extrakorporalen Blutkreislauf gefördert wird. Jedoch können zusätzlich andere Komponenten des Kreislaufs in die venöse Leitung eingebunden sein, die in Figur 1 aber nicht gezeigt sind. Über eine an der Abgabeseite der Pumpe angeschlossene arterielle Schlauchleitung 3 gibt die Zentrifugalpumpe 1 das Blut an den arteriellen Abschnitt des extrakorporalen Blutkreislaufs ab.

Auf der arteriellen Seite der Pumpe sind ein Durchflusssensor 5 und ein Drucksensor 6 vorgesehen. Der Durchflusssensor 5 dient zur Erfassung der Abgabemenge auf der Abgabeseite der Pumpe 1 und gibt ein der Abgabemenge entsprechendes Messsignal ab. Der Drucksensor 6 dient zur Erfassung des Druckes auf der Abgabeseite der Pumpe 1 und gibt ein der Durchflussmenge entsprechendes Messsignal ab. Bezüglich des Drucksensors 6 ist zu beachten, dass diese Messstelle unkritisch ist, da auf der arteriellen Seite der Pumpe ein Überdruck (gegenüber dem Atmosphärendruck) herrscht und es deshalb nicht zum Eindringen von Luft in die arterielle Schlauchleitung 3 sondern allenfalls zum Austreten von Blut aus der arteriellen Schlauchleitung 3 kommen kann, sofern eine Undichtigkeit im Bereich der Messstelle auftritt.

Wie Figur 1 weiter zeigt, umfasst das hier beschriebene Ausführungsbeispiel einer erfindungsgemäßen Vorrichtung ferner eine Auswerteeinrichtung 4, der das Messsignal des Durchflusssensors 5 und das Messsignal des Drucksensors 6 zugeführt werden. Außerdem wird der Auswerteeinrichtung ein weiteres Signal zugeführt, beispielsweise der Messwert eines Drehzahlsensors 1a oder das Ansteuersignal der Pumpe, das der Drehzahl der Zentrifugalpumpe 1 entspricht. Wie Figur 1 zeigt, werden bei dem hier beschriebenen Ausführungsbeispiel die Messsignale und das weitere Signal der Auswerteeinrichtung 4 über Signalleitungen 4a bis 4c zugeführt. Sofern die Auswerteeinrichtung 4 in eine Steuereinrichtung für die Ansteuerung der Pumpe integriert ist, ist die gesonderte Zuführung eines drehzahlabhängigen Signals nicht erforderlich. In Figur 1 ist diese Ausgestaltung dadurch angedeutet, dass eine (gestrichelt gezeichnete) Ansteuersignalleitung 4d vorgesehen ist, über die die in diesem Fall als Steuereinrichtung ausgelegte Auswerteeinrichtung 4 der Pumpe 1 ein Ansteuersignal zuführt und auf diese Weise die Drehzahl der Pumpe unmittelbar bestimmt.

In jedem Fall aber wertet die Auswerteeinrichtung 4 die Messsignale und die Drehzahl der Zentrifugalpumpe aus und ermittelt auf der Grundlage der Messsignale und der Drehzahl einen Wert, der dem Druck auf der Ansaugseite der Pumpe entspricht. Dabei wird ausgenutzt, dass der Druckanstieg (Gradient Auslassdruck-Einlassdruck) über einer Zentrifugalpumpe eine Funktion der Drehzahl und des Flusses ist, die zum Beispiel mit Hilfe eines Kennlinienfeldes beschrieben werden kann. Veränderter Widerstände auf der venösen oder der arteriellen Seite der Pumpe haben darauf keinen Einfluss. Somit kann bei bekanntem Druck auf der Abgabeseite, sowie bekanntem Fluss und bekannter Drehzahl der Druck auf der Ansaugseite ermittelt werden, indem zunächst der Druckanstieg über der Pumpe mit Hilfe des Kennlinienfelds (und gegebenenfalls Interpolation zwischen Stützwerten des Kennlinienfelds) bestimmt und danach der so bestimmte Druckanstieg von dem Druck auf der Abgabeseite der Pumpe subtrahiert wird.

Das Kennlinienfeld muss für einen bestimmten Zentrifugalpumpentyp zuvor messtechnisch ermittelt werden. Die Werte des Kennlinienfeldes werden in der Auswerteeinrichtung 4 vorzugsweise in einem Speicher 7 abgelegt, so dass die Auswerteeinrichtungen bei der Ermittlung des dem Druck auf der Ansaugseite der Pumpe entsprechenden Werts auf das Kennlinienfeld der Zentrifugalpumpe zugreifen kann, wobei die Messsignale der Sensoren auf der arteriellen Seite und die Drehzahl als Zugriffsparameter verwendet werden können. Sofern ein Stützwert bei einer gegebenen Parameterkombination im Kennlinienfeld nicht vorhanden ist, wird ein geeigneter Punkt im Kennlinienfeld mit Hilfe von (linearer) Interpolation bestimmt.

Wenn die Auswerteinrichtung 4 einen übermäßigen Unterdruck auf der venösen Seite der Zentrifugalpumpe ermittelt, kann über eine Anzeigeeinrichtung 8 ein Alarmhinweis an eine Bedienperson ausgegeben werden, die dann die geeigneten Gegenmaßnahmen trifft, um den Unterdruck in der venösen Leitung zu reduzieren. Die Auswerteeinrichtung 4 ist dazu über eine Signalleitung 4e mit der Anzeigeeinrichtung 8 verbunden. Mit Hilfe der Anzeigeeinrichtung 8 ist es ferner möglich, dass die Auswerteeinrichtung 4 kontinuierlich eine Druckanzeige zumindest für den Unterdruck auf der venösen Seite der Pumpe liefert, so dass die Bedienperson im laufenden Betrieb einen Überblick über die aktuellen Druckverhältnisse erhält.

Sofern wie oben beschrieben die Auswerteeinrichtung 4 auch die Funktion einer Steuereinrichtung übernimmt oder in eine Steuereinrichtung integriert ist, kann bei einem übermäßigen Unterdruck auf der venösen Seite der Zentrifugalpumpe direkt in den Betrieb der Pumpe eingegriffen und die Drehzahl abgesenkt werden, um den Unterdruck in der venösen Leitung zu verringern. Für die Zuführung eines entsprechenden Ansteuersignals zur Pumpe 1 kann die bereits angesprochene Ansteuersignalleitung 4d verwendet werden.

## Patentansprüche

1. Vorrichtung zur Förderung von Blut in einem extrakorporalen Kreislauf mit
- einer Zentrifugalpumpe, die das zu fördernde Blut über eine an der Ansaugseite der Pumpe angeschlossene venöse Schlauchleitung (2) ansaugt und über eine an der Abgabeseite der Pumpe angeschlossene arterielle Schlauchleitung (3) abgibt,
- einem Durchflusssensor (5), der zur Erfassung der Abgabemenge auf der Abgabeseite der Pumpe (1) vorgesehen ist und der ein der Abgabemenge entsprechendes Messsignal abgibt,
- einem Drucksensor (6), der zur Erfassung des Druckes auf der Abgabeseite der Pumpe (1) vorgesehen ist und der ein dem Druck entsprechendes Messsignal abgibt,
**gekennzeichnet durch**
- eine Auswerteeinrichtung (4), der das Messsignal des Durchflusssensors (5) und das Messsignal des Drucksensors (6) zugeführt werden und die unter Einbeziehung der beiden Messsignale und der Drehzahl der Zentrifugalpumpe einen dem Druck auf der Ansaugseite der Pumpe entsprechenden Wert ermittelt.

2. Vorrichtung nach Anspruch 1, bei der die Auswerteeinrichtung (4) bei der Ermittlung des dem Druck auf der Ansaugseite der Pumpe entsprechenden Werts auf ein Kennlinienfeld der Zentrifugalpumpe zugreift.

3. Vorrichtung nach Anspruch 2, bei der in der Auswerteeinrichtung (4) das Kennlinienfeld der Zentrifugalpumpe in einem Speicher (7) abgelegt ist.

4. Vorrichtung nach einem der Ansprüche 1 bis 3, bei der die Auswerteeinrichtung (4) mit einer Anzeigeeinrichtung (8) für die Anzeige zumindest des Unterdrucks auf der Ansaugseite der Pumpe (1) und/oder für die Alarmierung einer Bedienperson verbunden ist.

5. Vorrichtung nach einem der Ansprüche 1 bis 3, bei der der Auswerteeinrichtung (4) ein Messsignal eines Drehzahlsensors (1a) zugeführt wird.

6. Vorrichtung nach einem der Ansprüche 1 bis 3, bei der der Auswerteeinrichtung (4) ein Ansteuersignal der Pumpe (1) zugeführt wird, das der Drehzahl der Pumpe entspricht.

7. Vorrichtung nach einem der Ansprüche 1 bis 4, bei der die Auswerteeinrichtung (4) die Funktion einer Steuereinrichtung für die Zentrifugalpumpe (1) wahrnimmt oder in eine Steuereinrichtung für die Zentrifugalpumpe (1) integriert ist, so dass ein die Drehzahl der Zentrifugalpumpe (1) bestimmendes Ansteuersignal der Zentrifugalpumpe zuführbar ist.

8. Vorrichtung nach Anspruch 7, bei der die Auswerteeinrichtung (4) die Drehzahl der Pumpe (1) beim Auftreten eines vorgegebenen Wertes für den Druck auf der Ansaugseite der Pumpe reduziert.

## Claims

1. Apparatus for the transport of blood in an extracorporeal circuit, having
- a centrifugal pump which draws in the blood to be transported via a venous tube (2) connected to the intake side of the pump and delivers it via an arterial tube (3) connected to the delivery side of the pump,
- a flow sensor (5) which is provided for detecting the delivered quantity on the delivery side of the pump (1) and which emits a measurement signal corresponding to the delivered quantity,
- a pressure sensor (6) which is provided for detecting the pressure on the delivery side of the pump (1) and which emits a measurement signal corresponding to the pressure,
**characterised by**
- an evaluating device (4) to which the measurement signal of the flow sensor (5) and the measurement signal of the pressure sensor (6) are delivered, and which, taking into account the two measurement signals and the speed of rotation of the centrifugal pump, determines a value corresponding to the pressure on the intake side of the pump.

2. Apparatus according to claim 1, in which the evaluating device (4), on determining the value corresponding to the pressure on the intake side of the pump, accesses a family of characteristics of the centrifugal pump.

3. Apparatus according to claim 2, in which in the evaluating device (4) the family of characteristics of the centrifugal pump is filed in a memory (7).

4. Apparatus according to any of claims 1 to 3, in which the evaluating device (4) is connected to an indicator device (8) for indicating at least the partial pressure on the intake side of the pump (1) and/or for alerting an operator.

5. Apparatus according to any of claims 1 to 3, in which a measurement signal of a speed sensor (1a) is delivered to the evaluating device (4).

6. Apparatus according to any of claims 1 to 3, in which a drive signal for the pump (1) which corresponds to the speed of the pump is delivered to the evaluating device (4).

7. Apparatus according to any of claims 1 to 4, in which the evaluating device (4) performs the function of a control device for the centrifugal pump (1) or is integrated in a control device for the centrifugal pump (1), so that a drive signal which determines the speed of the centrifugal pump (1) can be delivered to the centrifugal pump.

8. Apparatus according to claim 7, in which the evaluating device (4) reduces the speed of the pump (1) when a predetermined value for the pressure occurs on the intake side of the pump.

## Revendications

1. Dispositif pour la circulation de sang dans un circuit extracorporel, comportant
- une pompe centrifuge qui aspire le sang à faire circuler par l'intermédiaire d'une conduite veineuse flexible (2) raccordée au côté d'aspiration de la pompe, et le refoule par l'intermédiaire d'une conduite artérielle flexible (3) raccordée au côté refoulement de la pompe,
- un capteur de débit (5) qui est prévu pour détecter le débit de refoulement côté refoulement de la pompe (1) et qui émet un signal de mesure correspondant au débit de refoulement,
- un capteur de pression (6) qui est prévu pour détecter la pression côté refoulement de la pompe (1) et qui émet un signal de mesure correspondant à la pression,
**caractérisé par**
- un dispositif d'analyse (4) auquel sont transmis le signal de mesure du capteur de débit (5) et le signal de mesure du capteur de pression (6), et qui détermine une valeur correspondant à la pression côté aspiration de la pompe en recourant aux deux signaux de mesure et à la vitesse de rotation de la pompe centrifuge.

2. Dispositif selon la revendication 1, où le dispositif d'analyse (4) recourt à un réseau de caractéristiques de la pompe centrifuge pour déterminer la valeur correspondant à la pression côté aspiration de la pompe.

3. Dispositif selon la revendication 2, où le réseau de caractéristiques de la pompe centrifuge est sauvegardé dans une mémoire (7) du dispositif d'analyse (4).

4. Dispositif selon l'une des revendications 1 à 3, où le dispositif d'analyse (4) est relié à un dispositif indicateur (8) destiné à indiquer au moins la dépression côté aspiration de la pompe (1) et/ou à alerter un opérateur.

5. Dispositif selon l'une des revendications 1 à 3, où un signal de mesure d'un capteur de vitesse de rotation (la) est transmis au dispositif d'analyse (4).

6. Dispositif selon l'une des revendications 1 à 3, où un signal de commande de la pompe (1) correspondant à la vitesse de rotation de la pompe est transmis au dispositif d'analyse (4).

7. Dispositif selon l'une des revendications 1 à 4, où le dispositif d'analyse (4) assume la fonction d'un dispositif de commande pour la pompe centrifuge (1) ou est intégré à un dispositif de commande pour la pompe centrifuge (1), si bien qu'un signal de commande déterminant la vitesse de rotation de la pompe centrifuge (1) peut être transmis à la pompe centrifuge.

8. Dispositif selon la revendication 7, où le dispositif d'analyse (4) ralentit la vitesse de rotation de la pompe (1) à l'apparition d'une valeur définie pour la pression côté aspiration de la pompe.
